# EUROPEAN PATENT SPECIFICATION

(11) **EP 2 687 149 B1**
(45) Date of publication and mention of the grant of the patent: **06.04.2016**
(21) Application number: 12176925.1
(22) Date of filing: 18.07.2012
(51) Int. Cl.: A61M 16/04, A61M 16/08

(54) **Tracheotomy tube assembly**
Tracheotomietubusanordnung
Ensemble de tube de trachéotomie

(43) Date of publication of application: 22.01.2014
(73) Proprietor: Vitaltec Corporation, Taichung City (TW)
(72) Inventor: Chiu, Sheng-Yu, Taichung City (TW); Chang, Ti-Li, Taichung City (TW)
(74) Representative: Becker Kurig Straus

(56) References cited:
- EP-A1- 1 479 405
- EP-A1- 2 452 714
- WO-A1-95/04566
- WO-A1-2007/038563
- US-A1- 2009 095 302
- US-A1- 2010 108 076
- US-A1- 2012 085 350

## Description

### 1. Field of the Invention

The present invention relates to a tracheotomy tube assembly, especially to a tracheotomy tube assembly that has simplified structure and is able to ease discomfort of a patient.

### 2. Description of the Prior Art(s)

When a patient is unable to breathe autonomously or when respiratory disorders occur, a doctor makes an incision on an anterior portion of the neck of the patient in order to put a tracheotomy tube into a trachea of the patient through the incision. Then the tracheotomy tube is connected to a peripheral instrument or a connecting tube to assist the patient with breathing or to allow the doctor to suck phlegm out of the trachea of the patient.

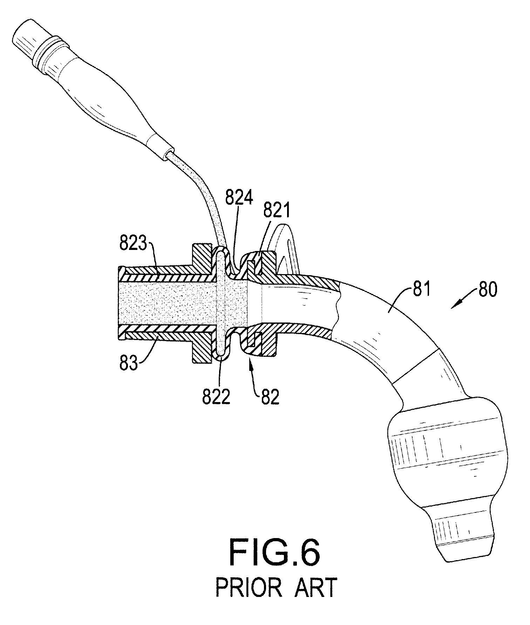

With reference to Fig. 6, a conventional tracheotomy tube assembly 80 comprises an insertion tube 81, a connector 82 and a reinforcing tube 83. The connector 82 is made of plastic, is tubular, soft and resilient, is connected to the insertion tube 81, and has two ends, a holding portion 821, a tubular portion 823, an annular protrusion 822 and an annular groove 824. The holding portion 821 is formed on one of the ends of the connector 82 and is securely connected to a proximal end of the insertion tube 81. The tubular portion 823 is formed on the other one of the ends of the connector 82. The annular protrusion 822 is formed around an outer surface of the connector 82 and is disposed between the holding portion 821 and the tubular portion 823. The annular groove 824 is defined between the holding portion 821 and the annular protrusion 822. The reinforcing tube 83 is made of plastic, is hard, and is securely mounted on and around the tubular portion 823 of the connector 82 to enhance structural strength of the tubular portion 823 of the connector 82. When the conventional tracheotomy tube assembly 80 is employed, the insertion tube 81 is inserted into the trachea of the patient, the connector 82 is disposed outside the trachea of the patient, and the reinforcing tube 83 is connected to a connecting tube or a peripheral instrument. Since the connector 82 is soft and resilient, the annular protrusion 822 and the annular groove 824 of the connector 82 can be twisted.

In addition, US 2010/108076 discloses a tracheostomy tube but does not disclose a connection arrangement for a resilient connector and a connecting sleeve. EP2452714 and US 2012/085350 respectively disclose a tracheostomy tube but do not disclose two wings integrally formed on a flexible connector. WO 95/04566 discloses a connector for locking an inner cannula inside an outer cannula but does not disclose a flexible connector connected with a connecting tube. US 2009/095302 discloses a speaking valve and does not disclose a flexible connector made of soft and resilient material. EP 1479405 discloses a one touch connector for tracheotomy tube but does not disclose a flexible connector with a connecting tube.

However, the reinforcing tube 83 and the connector 82 are two separate parts. Therefore, clearance exists between the reinforcing tube 83 and the tubular portion 823 of the connector 82. Consequently, the phlegm of the patient remains in the clearance and is hard to be cleaned so the patient is in danger of being infected. Moreover, mounting the reinforcing tube 83 on the tubular portion 823 of the connector 82 increases manufacturing processes and manufacturing time of the conventional tracheotomy tube assembly 80. Furthermore, since the reinforcing tube 83 and the annular protrusion 822 of the connector 82 do not rotate simultaneously, the reinforcing tube 83 and the annular protrusion 822 interfere with each other and the reinforcing tube 83 does not rotate smoothly.

The main objective of the present invention is to provide a tracheotomy tube assembly. The tracheotomy tube assembly has an insertion set, a flexible connector connected to an insertion tube of the insertion set, and a connecting tube. A proximal end of the connecting tube is connected to an annular connecting portion of the flexible connector that is disposed on a second end of the flexible connector. The insertion set has an insertion tube that has a distal end and a proximal end, and a balloon mounted on and around the insertion tube and disposed adjacent to the distal end of the insertion tube. The flexible connector is made of plastic, is soft and resilient, is connected to the proximal end of the insertion tube and has a holding portion, an annular connecting portion and an annular groove. The holding portion is formed on and around a first end of the flexible connector and is securely connected to the proximal end of the insertion tube. The annular connecting portion is formed on an outer surface of the flexible connector and is disposed around a second end of the flexible connector. The annular groove is defined between the annular connecting portion and the holding portion. The connecting tube is made of plastic, is hard and is connected to the annular connecting portion of the flexible connector. The annular connecting portion of the flexible connector has an engaging recess formed in the annular connecting portion. The connecting tube has a proximal end securely mounted in the engaging recess of the annular connecting portion of the flexible connector. The connecting tube further has an engaging wall formed on and around and radially protruding from the proximal end of the connecting tube, and securely mounted in the engaging recess of the annular connecting portion of the flexible connector. The flexible connector further has an engaging wall being annular, and formed on and axially protruding from the second end of the flexible connector. The connecting tube further has an engaging recess being annular, axially formed in the proximal end of the connecting tube and securely mounted around the engaging wall of the annular connecting portion of the flexible connector. The flexible connector further has two wings integrally formed on and protruding from the holding portion and disposed opposite to each other.

Overlapping portions of the connecting tube and the flexible connector are greatly reduced to prevent the patient from being infected. Movements of the connecting tube do not interfere with the insertion set, so discomfort of the patient can be eased. Structure of the flexible connector is simplified, so the tracheotomy tube assembly has less manufacturing processes and low manufacturing cost.

### IN THE DRAWINGS:

Fig. 1 is a perspective view of a tracheotomy tube assembly in accordance with the present invention;
Fig. 2 is an exploded perspective view of the tracheotomy tube assembly in Fig. 1;
Fig. 3 is a side view in partial section of the tracheotomy tube assembly in Fig. 1;
Fig. 4 is an operational side view of the tracheotomy tube assembly in Fig. 1;
Fig. 5 is an enlarged operational side view of the tracheotomy tube assembly in Fig. 1; and
Fig. 6 is a side view in partial section of a conventional tracheotomy tube assembly in accordance with the prior art.

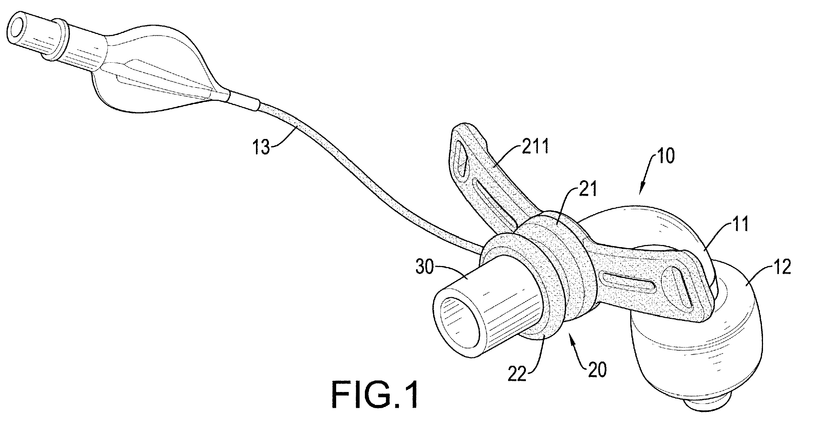

With reference to Fig. 1, a tracheotomy tube assembly in accordance with the present invention comprises an insertion set 10, a flexible connector 20 and a connecting tube 30.

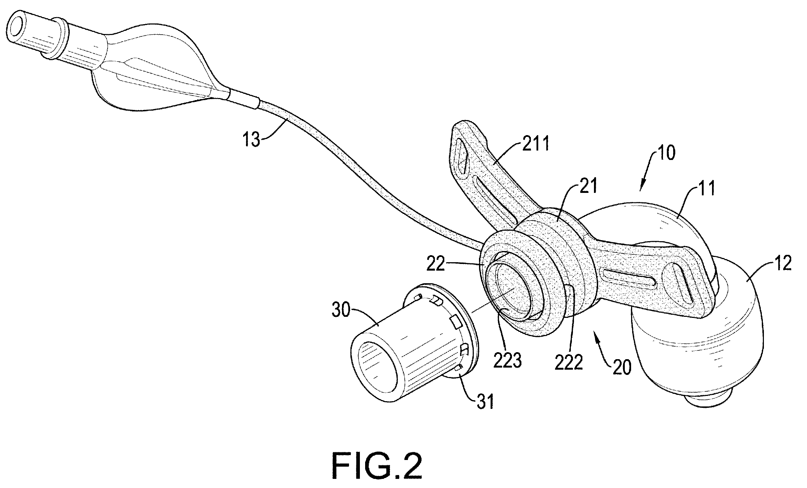

With further reference to Fig. 2, the insertion set 10 has an insertion tube 11, a balloon 12 and a puffing tube 13. The insertion tube 11 has a distal end and a proximal end. The balloon 12 is mounted on and around the insertion tube 11 and is disposed adjacent to the distal end of the insertion tube 11. The puffing tube 13 is connected to and communicates with the balloon 12.

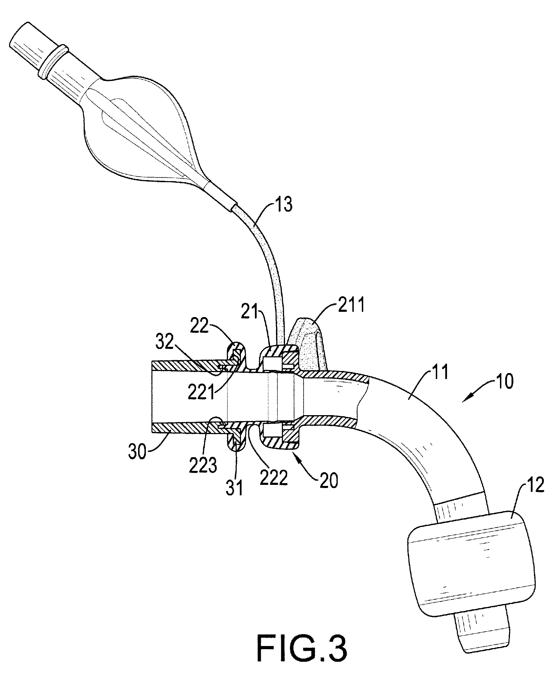

With further reference to Fig. 3, the flexible connector 20 is made of plastic, is soft and resilient, is connected to the proximal end of the insertion tube 11 and has a first end, a second end, an outer surface, a holding portion 21, two wings 211, an annular connecting portion 22, an engaging wall 223 and an annular groove 222. The holding portion 21 is formed on and around the first end of the flexible connector 20, and is securely connected to the proximal end of the insertion tube 11. The wings 211 are integrally formed on and protrude from the holding portion 21 and are disposed opposite to each other. The annular connecting portion 22 is formed on the outer surface of the flexible connector 20, is disposed around the second end of the flexible connector 20 and has an engaging recess 221 formed in the annular connecting portion 22. The engaging wall 223 is annular, and is formed on and axially protrudes from the second end of the flexible connector 20. The annular groove 222 is defined between the annular connecting portion 22 and the holding portion 21.

The connecting tube 30 is made of plastic, is hard, is connected to the annular connecting portion 22 of the flexible connector 20 and has a proximal end, an engaging wall 31 and an engaging recess 32. The proximal end of the connecting tube 30 is securely mounted in the engaging recess 221 of the annular connecting portion 22. The engaging wall 31 of the connecting tube 30 is formed on and around and radially protrudes from the proximal end of the connecting tube 30, and is securely mounted in the engaging recess 221 of the annular connecting portion 22 of the flexible connector 20. The engaging recess 32 of the connecting tube 30 is annular, is axially formed in the proximal end of the connecting tube 30 and is securely mounted around the engaging wall 223 of the annular connecting portion 22 of the flexible connector 20.

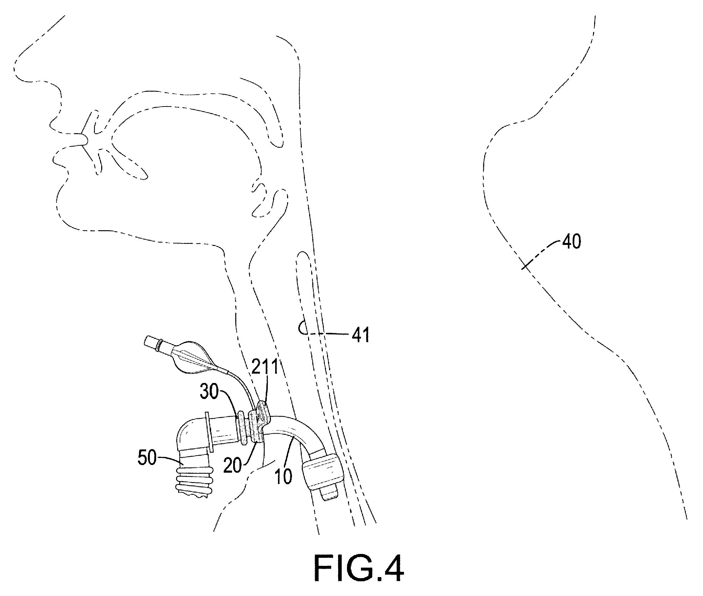

With further reference to Fig. 4, when the tracheotomy tube assembly is employed, the insertion tube 11 is inserted into a trachea 41 of a patient 40 through an incision on the neck of the patient 40. Then the balloon 12 is inflated via the puffing tube 13 and is settled in the trachea 41. A harness is connected to the wings 211 of the flexible connector 20 for fastening the tracheotomy tube assembly to the neck of the patient 40.

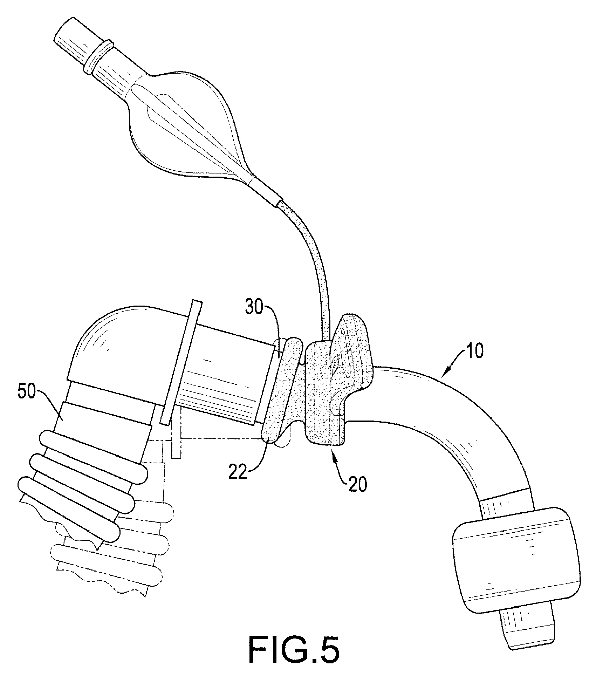

With further reference to Fig. 5, the connecting tube 30 is connected to a peripheral connecting tube 50 or a peripheral instrument. When the peripheral connecting tube 50 and the connecting tube 30 are moved or rotated, the annular groove 222 of the annular connecting portion 22 of the flexible connector 20 is twisted accordingly. Movements of the connecting tube 30 do not interfere with the insertion set 10 so discomfort of the patient 40 can be eased.

The tracheotomy tube assembly as described has the following advantages. Since the connecting tube 30 and the flexible connector 20 are connected to each other via the proximal end of the connecting tube 30 and the annular connecting portion 22 that is disposed on the second end of the flexible connector 20, overlapping portions of the connecting tube 30 and the flexible connector 20 are greatly reduced. Consequently, phlegm or juice of the patient 40 that remains in clearance between the connecting tube 30 and the flexible connector 20 is also greatly reduced to prevent the patient 40 from being infected. Furthermore, structure of the flexible connector 20 is simplified, so the tracheotomy tube assembly has less manufacturing processes and low manufacturing cost.

## Claims

1. A tracheotomy tube assembly comprising an insertion set (10) having an insertion tube (11) having a distal end and a proximal end, and a balloon (12) mounted on and around the insertion tube (11) and disposed adjacent to the distal end of the insertion tube (11), a flexible connector (20) made of plastic being soft and resilient, connected to the proximal end of the insertion tube (11), and having a holding portion (21) formed on and around a first end of the flexible connector (20) and securely connected to the proximal end of the insertion tube (11), an annular connecting portion (22) formed on an outer surface of the flexible connector (20) and disposed around a second end of the flexible connector (20), and an annular groove (222) defined between the annular connecting portion (22) and the holding portion (21), and a connecting tube (30) made of plastic being hard and connected to the annular connecting portion (22) of the flexible connector (20), wherein
the annular connecting portion (22) of the flexible connector (20) has an engaging recess (221) formed in the annular connecting portion (22);
the connecting tube (30) has a proximal end securely mounted in the engaging recess (221) of the annular connecting portion (22) of the flexible connector (20);
the connecting tube (30) further has an engaging wall (31) formed on and around and radially protruding from the proximal end of the connecting tube (30), and securely mounted in the engaging recess (221) of the annular connecting portion (22) of the flexible connector (20);
the flexible connector (20) further has an engaging wall (223) being annular, and formed on and axially protruding from the second end of the flexible connector (20);
the connecting tube (30) further has an engaging recess (32) being annular, axially formed in the proximal end of the connecting tube (30) and securely mounted around the engaging wall (223) of the annular connecting portion (22) of the flexible connector (20), the tracheotomy tube assembly being **characterized in that**:
the flexible connector (20) further has two wings (211) integrally formed on and protruding from the holding portion (21) and disposed opposite to each other.

## Patentansprüche

1. Tracheotomie-Röhrenbaugruppe, umfassend einen Einbringsatz (10) mit einer Einbringröhre (11), die ein distales Ende und ein proximales Ende aufweist, und einen Ballon (12), der an und um die Einbringröhre (11) herum angebracht ist und der benachbart zu dem distalen Ende der Einbringröhre (11) angeordnet ist, ein flexibles Verbindungselement (20), das aus Kunststoff hergestellt ist, der weich und biegsam ist, verbunden mit dem proximalen Ende der Einbringröhre (11), und mit einem Halteabschnitt (21), der an und um ein erstes Ende des flexiblen Verbindungselements (20) herum angebracht ist und sicher mit dem proximalen Ende der Einbringröhre (11) verbunden ist, einen ringförmigen Verbindungsabschnitt (22), der an einer Außenfläche des flexiblen Verbindungselements (20) gebildet und um ein zweites Ende des flexiblen Verbindungselements (20) herum angeordnet ist, und eine ringförmige Nut (222), die zwischen dem ringförmigen Verbindungsabschnitt (22) und dem Halteabschnitt (21) definiert ist, und eine Verbindungsröhre (30), die aus Kunststoff hergestellt ist, der hart ist, und die mit dem ringförmigen Verbindungsabschnitt (22) des flexiblen Verbindungselements (20) verbunden ist,
wobei der ringförmige Verbindungsabschnitt (22) des flexiblen Verbindungselements (20) eine Eingriffsvertiefung (221) aufweist, die in dem ringförmigen Verbindungsabschnitt (22) gebildet ist;
wobei die Verbindungsröhre (30) ein proximales Ende aufweist, das sicher an der Eingriffsvertiefung (221) des ringförmigen Verbindungsabschnitts (22) des flexiblen Verbindungselements (20) angebracht ist;
wobei die Verbindungsröhre (30) weiterhin eine Eingriffswand (31) aufweist, die an und um das proximale Ende der Verbindungsröhre (30) herum gebildet ist und radial von dem proximalen Ende der Verbindungsröhre (30) absteht, und sicher in der Eingriffsvertiefung (221) des ringförmigen Verbindungsabschnitts (22) des flexiblen Verbindungselements (20) angebracht ist;
wobei das flexible Verbindungselement (20) weiterhin eine Eingriffswand (223) aufweist, die ringförmig ist und die an und axial abstehend von dem zweiten Ende des flexiblen Verbindungselements (20) gebildet ist;
wobei die Verbindungsröhre (30) weiterhin eine Eingriffsvertiefung (32) aufweist, die ringförmig, axial in dem proximalen Ende der Verbindungsröhre (30) gebildet und sicher um die Eingriffswand (223) des ringförmigen Verbindungsabschnitts (22) des flexiblen Verbindungselements (20) herum angebracht ist,
wobei die Tracheotomie-Röhrenbaugruppe **dadurch gekennzeichnet ist, dass**:
das flexible Verbindungselement (20) weiterhin zwei Flügel (211) aufweist, die integral an dem Halteabschnitt (21) gebildet sind und von diesem abstehen, und die einander gegenüberliegend angeordnet sind.

## Revendications

1. Montage de tube pour trachéotomie comprenant un set d'insertion (10) présentant un tube d'insertion (11) ayant une extrémité distale et une extrémité proximale, et un ballonnet (12) monté sur et autour du tube d'insertion (11) et disposé adjacent à l'extrémité distale du tube d'insertion (11), un connecteur flexible (20) en plastique, souple et résilient, relié à l'extrémité proximale du tube d'insertion (11), et présentant une partie de support (21) formée sur et autour d'une première extrémité du connecteur flexible (20) et reliée fixement à l'extrémité proximale du tube d'insertion (11), une partie de connexion annulaire (22) formée sur une surface extérieure du connecteur flexible (20) et disposée autour d'une seconde extrémité du connecteur flexible (20), et une rainure annulaire (222) définie entre la partie de connexion annulaire (22) et la partie de support (21), et un tube de connexion (30) en plastique dur et relié à la partie de connexion annulaire (22) du connecteur flexible (20), sachant que
la partie de connexion annulaire (22) du connecteur flexible (20) présente un creux d'engagement (221) formé dans la partie de connexion annulaire (22) ;
le tube de connexion (30) présente une extrémité proximale montée fixement dans le creux d'engagement (221) de la partie de connexion annulaire (22) du connecteur flexible (20) ;
le tube de connexion (30) présente en outre une paroi d'engagement (31) formée sur et autour, et en saillie radiale de, l'extrémité proximale du tube de connexion (30), et montée fixement dans le creux d'engagement (221) de la partie de connexion annulaire (22) du connecteur flexible (20) ;
le connecteur flexible (20) présente en outre une paroi d'engagement (223) annulaire, et formée sur et faisant saillie axialement de la seconde extrémité du connecteur flexible (20) ;
le tube de connexion (30) présente en outre un creux d'engagement (32) annulaire, formé axialement dans l'extrémité proximale du tube de connexion (30) et monté fixement autour de la paroi d'engagement (223) de la partie de connexion annulaire (22) du connecteur flexible (20), le tube pour trachéotomie étant **caractérisé en ce que** :
le connecteur flexible (20) présente en outre deux ailettes (211) formées intégralement sur et faisant saillie de la partie de support (21) et disposées de façon opposée l'une à l'autre.
